# EUROPÄISCHE PATENTSCHRIFT

(11) **EP 0 633 249 B1**
(45) Veröffentlichungstag und Bekanntmachung des Hinweises auf die Patenterteilung: **12.02.1997**
(21) Anmeldenummer: 94106175.6
(22) Anmeldetag: 21.04.1994
(51) Int. Cl.: C07D 213/79, C07D 213/84

(54) **Verfahren zur Herstellung von 2,4-Pyridindicarbonsäure**
Process for the preparation of 2,4-pyridinedicarboxylic acid
Procédé pour la préparation d'acide pyridinedicarboxyliques-2,4

(30) Priorität: 10.07.1993 DE 4323071
(43) Veröffentlichungstag der Anmeldung: 11.01.1995
(73) Patentinhaber: Weyl GmbH, 68305 Mannheim (DE)
(72) Erfinder: Sendelbach, Stefan, Dr., D-74172 Neckarsulm (DE); Orth, Winfried, Dr., D-67454 Hassloch (DE); Weiss, Wolfgang, Dr., D-68535 Edingen-Neckarhausen (DE); Kleffner, Hans Werner, Dr., D-67271 Battenberg (DE)
(74) Vertreter: Patentanwälte Zellentin & Partner

(56) Entgegenhaltungen:
- CHEMICAL ABSTRACTS, vol. 55, no. 7, 1961, Columbus, Ohio, US; abstract no. 6478b, H. TANI 'Reaction of N-alkoxypyridinium derivatives.'
- JOURNAL OF THE CHEMICAL SOCIETY, CHEMICAL COMMUNICATIONS., Nr.5, 1990, LETCHWORTH GB Seiten 370 - 371 C.W. SOMAWARDHANA ET AL. 'Novel solid-state support for radio synthesis of radiopharmaceuticals labelled with [11C]-cyanide.'
- BULLETIN OF THE CHEMICAL SOCIETY OF JAPAN, Bd.43, Nr.10, 1970, TOKYO JP Seiten 3210 - 3214 E. MATSUMURA ET AL. 'Studies of the Reissert-Kaufmann type reaction of 4-nitropyridine N-oxyde and its homologues.'
- R.A. ABRAMOVITCH 'Chemistry of heterocyclic compounds. Vol. 14: Pyridine and its derivatives. Supplement part 3.' 1974 , WILEY , NEW YORK * Seite 300 *
- CHEMICAL AND PHARMACEUTICAL BULLETIN, Bd.7, 1959, TOKYO JP Seiten 930 - 934 H. TANI 'The reaction of N-alkoxypyridinium derivatives.'

## Beschreibung

Die Erfindung betrifft ein Verfahren zur Herstellung von 2-Cyanoisonicotinamid und 2,4-Pyridindicarbonsäure aus Isonicotinsäureamid, wobei als Zwischenprodukt Isonicotinamid-N-oxid hergestellt wird.

Beide Verbindungen stellen wertvolle Zwischenprodukte für die Synthese pharmazeutischer Wirkstoffe dar. Beispielsweise wird 2,4-Pyridindicarbonsäure zur Herstellung von Arzneimitteln zur Hemmung der Prolin- und Lysinhydroxylase oder als Zwischenprodukt für Fibrosuppressiva und Immunsuppressiva verwendet. 2-Cyanoisonicotinamid wiederum läßt sich als Zwischenprodukt zur Herstellung von 2,4-Pyridindicarbonsäure verwenden.

Aus der Literatur sind verschiedene Methoden zur Herstellung von 2,4-Pyridindicarbonsäure bekannt. Beispielsweise erfolgt die Bildung der Dicarbonsäure aus 2,4-Dimethylpyridin beim Erhitzen mit SeO₂ in 3-Methylpyridin bei 110 °C oder in Essigsäureethylester, Methanol oder Xylol unter Rückfluß, wobei eine Ausbeute von 67 % erzielt werden kann (Jerchel et al.; Liebigs Annalen der Chemie, 613 (1958), 153, 155, 162; Marcot, B.; Palland, R.; Compt. rend. 248 (1959), 252 - 4).

Auch beim Erhitzen von 2,4-Dimethylpyridin in Gegenwart von Selen und konzentrierter Schwefelsäure wird die Dicarbonsäure gebildet, jedoch nur in einer theoretischen Ausbeute von 29 % (Ochiai, E.; Okuda, S.; J. Pharm. Soc. Japan 70 1950, 156 - 61).

Nach heutigen Reinheitsanforderungen, die an Zwischenprodukte zur Herstellung von pharmazeutischen Wirkstoffen gestellt werden, sind diese Methoden jedoch nicht durchführbar, da auch nach aufwendigen Reinigungsmaßnahmen immer noch Spuren des verwendeten toxischen Selens oder Selendioxids im Produkt verbleiben.

Zwar kann durch Oxidation von 2,4-Dimethylpyridin mit konzentrierter Salpetersäure bei 185 bis 190 °C unter Druck 2,4-Pyridindicarbonsäure in einer Ausbeute von 65 % erzielt werden, jedoch ist diese Methode nicht in einfacher Weise im technischen Maßstab durchführbar, da die Oxidation aufgrund der starken Reaktivität in 2,4-Position alkylierter Pyridine nur unter kontrollierten Bedingungen durchgeführt werden kann, und die Bildung nitroser Gase besondere Sicherheitsmaßnahmen erfordert.

Als weitere Methode wird in der Patentschrift DBP 10 10 524 eine Oxidation mit Sauerstoff in Gegenwart äquimolarer Mengen Kupfernitrat in wäßriger Lösung bei 225 bis 230 °C und 60 at beschrieben. Die durch die Oxidation gebildete Dicarbonsäure fällt dabei als Kupfersalz aus. Die Freisetzung der Säure erfolgt über die Bildung des Natriumsalzes, das anschließend mit Salzsäure zersetzt wird. Zwar fällt bei dieser Methode das Kupfersalz erst in verhältnismäßig hoher Ausbeute an, durch die zweistufige Freisetzung der Säure geht jedoch wiederum Produkt verloren, und, was für die weitere Verwendung für pharmazeutische Produkte von Bedeutung ist, es bleiben trotz mehrmaligen Umkristallisierens Spuren von Kupfer im Produkt erhalten, so daß diese Methode für diesen Zweck als nicht geeignet erscheint.

Auch die Freisetzung der Säure mit Hilfe von Schwefelwasserstoff unter Bildung schwerlöslichen Kupfersulfids, wie es verschiedentlich beschrieben worden ist, eignet sich nicht, da dem erhaltenen Produkt einerseits noch H₂S anhaftet, andererseits das Arbeiten mit H₂S aufgrund seiner Toxizität besondere Sicherheitsvorkehrungen bedarf.

Die Oxidation von 2,4-Dimethylpyridin mit Kaliumpermanganat führt zur Bildung von großen Mengen schwer entsorgbaren Braunsteins und ist wegen geringer Ausbeuten unwirtschaftlich (H. Meyer, H. Tropsch, Monatshefte Chemie 35 (1915), 189).

Es besteht daher die Aufgabe, ein neues, wirtschaftliches Verfahren zur Verfügung zu stellen, wodurch sich 2,4-Pyridincarbonsäure in hoher Ausbeute und Reinheit darstellen läßt, so daß es ohne aufwendige Reinigungsverfahren als Zwischenprodukt zur Herstellung pharmazeutischer Produkte verwendet werden kann.

Die Lösung der Aufgabe erfolgt durch ein Verfahren gemäß Anspruch 1 und seiner besonderen Ausgestaltung gemäß der Ansprüche 2 bis 6, das Zwischenprodukt 2-Cyanoisonicotinamid und gemäß Anspruch 7 und ihre Verwendung zur Herstellung von 2,4-Pyridindicarbonsäure gemäß Anspruch 8.

Da bislang durch bekannte Oxidationsmethoden 2,4-Pyridindicarbonsäure entweder nur in niedrigen Ausbeuten oder auch nach hohem Reinigungsaufwand nur in nicht ausreichender Reinheit, bzw. nur unter aufwendigen Sicherheitsvorkehrungen zu erhalten war, wurde nach einem alternativen, einfach durchzuführenden preiswerten Syntheseweg gesucht, wodurch 2,4-Pyridindicarbonsäure in hoher Ausbeute und hoher Reinheit hergestellt werden kann, so daß es als Zwischenprodukt für die Herstellung pharmazeutischer Wirkstoffe verwendet werden kann.

Aufgrund der zurückgehenden Stahlproduktion ist auf dem Markt eine Verknappung des indirekt dabei anfallenden Teers und somit auch für die daraus bisher durch Destillation gewonnenen Alkylpyridine, insbesondere für das 2,4-Dimethylpyridin eingetreten. Daher bieten sich als Ausgangsverbindungen zur Synthese von 2,4-Pyridindicarbonsäure preiswerte, durch Cyclisierung und evtl. anschließende Reaktionen erhaltene käufliche Pyridinderivate, wie 4-Pyridincarbonsäure, auch Isonicotinsäure genannt, 4-Pyridincarbonitril oder 4-Pyridincarboxamid, im folgenden Isonicotinamid genannt, an.

Die Bildung der 2,4-Pyridindicarbonsäure kann theoretisch aus diesen Verbindungen nach folgendem Schema erfolgen:

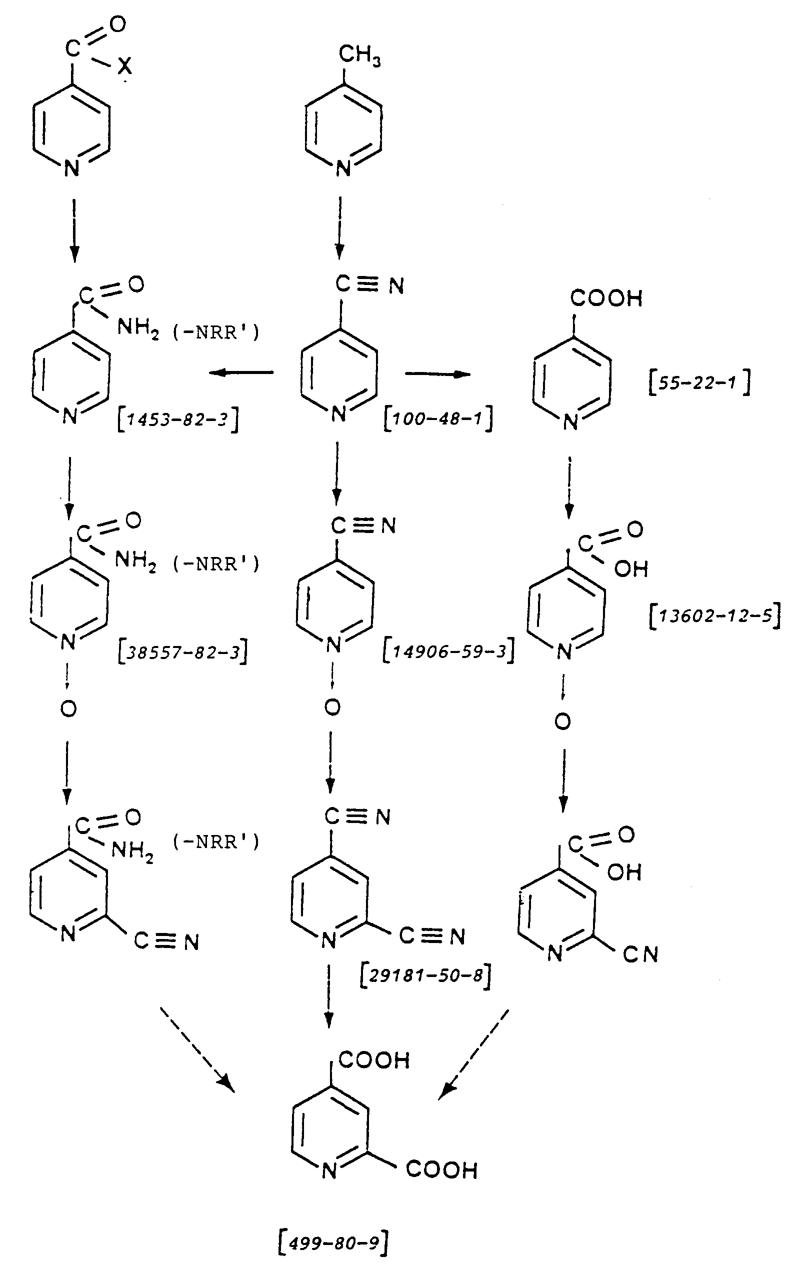

Aus Chemical Abstracts 55: 6478b ist bekannt, Isonicotinsäuremethylester-N-oxid in 2-Stellung zu cyanisieren, den resultierenden 2-Cyanoisonicotinsäuremethylester mit Alkali zu behandeln und das Reaktionsgemisch anschließend zu verestern. Der Fachmann könnte versuchen, bei diesem Reaktionsweg die Veresterungsstufe einzusparen und statt dessen die im Reaktionsgemisch erwartete 2,4-Pyridindicarbonsäure aufzuarbeiten. Jedoch läßt dies keine allzu hohe Ausbeute erwarten, da bereits die erste Teilreaktion, die Cyanierung, trotz molarem Überschuß an Cyanid nur eine Ausbeute des Zwischenproduktes von 69 % ergibt.

Überraschenderweise zeigte sich, daß sich aus dem kommerziell verfügbaren Isonicotinamid 2,4-Pyridindicarbonsäure in hohen Ausbeuten und hoher Reinheit gewinnen läßt, ohne daß die gebildeten Zwischenprodukte aufwendig gereinigt müssen.

Versucht man dagegen die Dicarbonsäure aus dem 4-Pyridincarbonitril herzustellen, ist bereits die Synthese des Zwischenproduktes Pyridin-2,4-dicarbonitril mit der Bildung eines hohen Anteils teerartiger Nebenprodukte verbunden, so daß aufwendige Reinigungsstufen zwischengeschaltet werden müssen und die Ausbeute letztendlich für die Durchführung im technischen Maßstab zu niedrig ist.

Die Darstellung aus Isonicotinsäure ist Gegenstand einer separaten Anmeldung und soll nicht berücksichtigt werden.

Ausgangsprodukt für das erfindungsgemäße Verfahren ist Isonicotinsäureamid. Zur Herstellung der 2,4-Pyridindicarbonsäure aus Isonicotinamid kann der aromatische Ring am Stickstoffatom nach bekannter, in der Literatur beschriebener Methode oxidiert werden. In hohen Ausbeuten kann dies z. B. in Gegenwart von Wasserstoffperoxyd in Essigsäure (Liberman et al., Bull. Soc. Chim. France 1958, Seiten 694 und 698), oder in Peressigsäure erfolgen. Das dabei anfallende Isonicotinamid-N-oxid wird ohne weitere Reinigung nach einer in US 2 991 285 beschriebenen Methode mit einem Alkylierungsmittel zum N-Alkoxy-isonicotinamidsalz umgesetzt. Als Alkylierungsmittel sind die gängigen Alkylierungsmittel einsetzbar, insbesondere Dimethyl- und Diethylsulfat.

Die Umsetzung kann in einem inerten Lösungsmittel, wie z. B. Toluol, aber auch ohne Zusatz eines Lösungsmittels bei einer Temperatur im Bereich von 90 bis 105 °C erfolgen.

Nach beendeter Reaktion kann das gebildete Pyridiniumsalz, welches durch spektroskopische Methoden, wie z. B. NMR, eindeutig charakterisiert werden kann, direkt zum 2-Cyanoisonicotinamid umgesetzt werden. Zu diesem Zweck wird das Salz in Wasser aufgenommen und mit einer wäßrigen Alkalicyanidlösung, bevorzugt einer Natrium- oder Kaliumcyanidlösung, vermischt. Die Umsetzung verläuft problemlos bei einer Temperatur oberhalb 35 °C, bevorzugt im Bereich von 20 bis 30 °C.

Ist das Pyridiniumsalz ohne Zusatz von Lösungsmitteln hergestellt worden, kann die Alkalicyanidlösung auch zum Pyridiniumsalz oder zu einer hergestellten wäßrigen Lösung gegeben werden. Das 2-Cyanoisonicotinamid fällt aus dieser Reaktionslösung als beigefarbener bis weißer Feststoff aus und kann durch einfache mechanische Methoden abgetrennt werden. Entsprechende Alkylamide fallen entweder als Festsubstanzen oder als Öle an.

Überraschenderweise wird das 2-Cyanoisonicotinamid auf diese Weise ohne Aufreinigung in einer Reinheit von etwa 98 % und in der hohen theoretischen Ausbeute von mehr als 90 %, bezogen auf das eingesetzte Isonicotinamid-N-oxid erhalten. Dieses ist besonders deshalb überraschend, weil vergleichbare Umsetzungen von 4-Cyanopyridin zum 2,4-Dicyanopyridin mit aufwendigen Reinigungsoperationen der Zwischenprodukte zur Abtrennung von teerartigen Nebenprodukten verbunden sind, und die Ausbeuten dementsprechend niedrig liegen. Außerdem werden bei vergleichbaren Umsetzungen das Alkylierungsmittel und das Alkalicyanid in hohem Überschuß unter Schutzgasatmosphäre benötigt, während bei der Herstellung des 2-Cyanoisonicotinamids äquimolare Mengen derselben genügen und die Reaktionsbedingungen milde sind.

So wird z. B. durch die Umsetzung von 4-Cyanopyridin-1-oxid mit Dimethylsulfat das Pyridiniumsalz als dickes, dunkles Öl erhalten, was eine Abtrennung der entstandenen Nebenprodukte vor der weiteren Umsetzung erforderlich macht. Durch die danach erfolgende Reaktion kann das 2,4-Dicyanopyridin nur in einer theoretischen Ausbeute von 54 % erhalten werden.

Die Hydrolyse des in der Literatur bisher nicht beschriebenen 2-Cyanoisonicotinamids zur 2,4-Pyridindicarbonsäure läßt sich in einer Ausbeute von mindestens 85 % in einfacher Weise durch dem Fachmann bekannte alkalische oder saure Verseifung und anschließende Einstellung des pH-Wertes der Reaktionslösung auf einen Wert im Bereich von 1 bis 4, bevorzugt von 2 bis 3 durchführen. Besonders bevorzugt sind für die saure Verseifung Schwefelsäure und Salzsäure, für die alkalische Verseifung Natron- und Kalilauge. Die 2,4-Pyridindicarbonsäure wird bei einem pH von 1 bis 4, bevorzugt 2 bis 3 gefällt.

Besonders vorteilhaft ist die saure Verseifung mit Schwefelsäure und anschließende pH-Einstellung mittels Ammoniak, da man auf diese Weise eine völlig aschefreie 2,4-Pyridindicarbonsäure erhält.

Vorteilhaft ist aber auch die Verseifung mit Natron- oder Kalilauge und anschließende pH-Einstellung mit Salzsäure, da man auf diese Weise ein sulfat- und ammoniumhaltiges Abwasser vermeidet.

Obwohl also die Herstellung der 2,4-Pyridindicarbonsäure z.B. aus dem käuflichen Isonicotinamid in einer vierstufigen Reaktion verläuft, wird die Pyridindicarbonsäure in einer Ausbeute von mindestens 70 %, bezogen auf die Ausgangsverbindung und einer Reinheit von 99 %, erhalten, ohne die Zwischenprodukte oder das Endprodukt aufwendig reinigen, bzw. alle Zwischenprodukte aus den Reaktionslösungen abtrennen zu müssen. Gleichzeitig können durch diese Herstellungsmethode Abwasserprobleme vermieden werden, da die Reaktionspartner in äquimolaren Mengen eingesetzt werden können, bzw. in so geringen Überschüssen eingesetzt werden, daß sie keine Probleme bereiten.

Im Gegensatz zu anderen Wegen sind in diesem Fall keine aufwendigen Sicherheitsmaßnahmen erforderlich, da unter milden Reaktionsbedingungen gearbeitet werden kann und die entstehenden Zwischen- und Nebenprodukte unbedenklich sind.

### BEISPIELE

### Beispiel 1

### Isonicotinamid-N-oxid

500 g (4,1 Mol) Isonicotinamid werden bei 80 bis 90 °C in 1 000 ml 85 %iger Essigsäure mit 170 ml (4,5 Mol) 70 %igem Wasserstoffperoxid versetzt. Man läßt über Nacht rühren und destilliert anschließend die Essigsäure unter Zuhilfenahme von Xylol ab. Der Rückstand wird in wenig Wasser aufgenommen, abgesaugt und getrocknet. Ausbeute 452 g (80 %) Isonicotinamid-N-oxid.

### Beispiel 2

### 2-Cyanoisonicotinamid

69 g (0,5 Mol) Isonicotinamid-N-oxid werden mit 50 ml (0,525 Mol) Dimethylsulfat versetzt und unter Rühren ca. 1,5 Stunden bei 95 bis 105 °C gerührt. Nach dem Abkühlen versetzt man mit 150 ml Wasser und tropft die Lösung innerhalb einer Stunde bei 20 bis 30 °C zu einer Lösung von 25,8 g Natriumcyanid in 100 ml Wasser. Nach beendeter Zutropfung läßt man noch eine halbe Stunde weiterrühren, saugt anschließend den Feststoff ab und wäscht mit Wasser nach. Trocknen im Trockenschrank bei 90 °C im Vakuum. Ausbeute 66,9 g (91 %) 2-Cyanoisonicotinamid.
¹H-NMR (200 MHz, DMSO-d₆) : δ = 7,98 (bereits s, 1H, CONH₂); 8,10 (m, 1H, 4-H); (d, ⁴J_{3.5} = 0,7 Hz, 1H, 4-H); 8,41 (bereits s, 1H, CONH₂); 8,91 (d, ³J_{5.6} = 5,1 Hz, 1H, 6-H).
IR (KBr) 3400 (s), 3320 (sh), 3150 (s), [cm⁻¹] 3090 (w), 2250 (-c≡ N), 1700 (C=O)
Schmp. 224.7 bis 229.2 °C (H₂O)

### Beispiel 3

### 2-Cyanoisonicotinamid

Zu 69 g (0,5 Mol) Isonicotinamid-N-oxid gibt man bei Raumtemperatur 65 g (0,55 Mol) Dimethylsulfat und erwärmt für 30 Minuten auf 90 bis 100 °C. Es entsteht ein Öl. Das erkaltete Öl wird in 75 ml Wasser aufgenommen und zu einer Lösung von 27 g (o,55 Mol) Natriumcyanid in 100 ml Wasser bei 30 °C zugetropft. Das Produkt fällt aus und wird abgesaugt, danach mit Wasser gewaschen. Trocknen im Vakuum bei 70 °C, ergibt 65 g (88 %) 2-Cyanoisonicotinamid.

### Beispiel 4

### 2,4-Pyridindicarbonsäure

147 g (1 Mol) 2-Cyanoisonicotinamid werden in 250 ml 80 bis 90 %iger Schwefelsäure 5 Stunden bei 100 °C gerührt. Anschließend wird unter Kühlung mit Ammoniak versetzt bis das Gemisch einen pH-Wert von etwa 2 hat und die Pyridin-2,4-dicarbonsäure ausfällt. Man saugt ab und wäscht mit kaltem Wasser nach. Trocknung im Trockenschrank bei 110 °C. Ausbeute 153,5 g (92 %) Pyridin-2,4-dicarbonsäure.
Schmp. 246 °C (Lit. 248 bis 250 °C)

### Beispiel 5

### 2,4-Pyridindicarbonsäure

73,6 g (0,5 Mol) 2-Cyanoisonicotinamid werden in einer Lösung von 126 g (2,25 Mol) Kaliumhydroxid in 300 ml Wasser 5 Stunden zum Rückfluß erhitzt. Nach dem Abkühlen wird mit 150 ml Wasser versetzt und mit konzentrierter Salzsäure ein pH von 2 eingestellt. Die ausgefallene Säure wird abgesaugt, gewaschen und bei 120 °C getrocknet. Ausbeute 73 g (82 %) 2,4-Pyridindicarbonsäure. Sulfatasche 0,27 %, Gehalt 98,8 %.

## Patentansprüche

1. Verfahren zur Herstellung von 2,4-Pyridindicarbonsäure, **dadurch gekennzeichnet**, daß
a) Isonicotinsäureamid zum entsprechenden N-oxid oxidiert,
b) Isonicotinamid-N-oxid in Gegenwart eines Alkylierungsmittels zum entsprechenden N-Alkoxyisonicotinamidsalz umgesetzt,
c) das Salz durch ein Alkalicyanid in wäßriger Lösung zum 2-Cyanoisonicotinamid umgesetzt und,
d) aus diesem Produkt durch Hydrolyse und anschließende pH-Einstellung auf einen pH-Wert im Bereich von 1 bis 4 2,4-Pyridindicarbonsäure in hochreiner Form gewonnen wird.

2. Verfahren gemäß Anspruch 1, **dadurch gekennzeichnet**, daß das Isonicotinsäureamid in Gegenwart von Wasserstoffperoxid in Essig- oder Peressigsäure zum entsprechenden N-oxid oxidiert wird.

3. Verfahren gemäß Anspruch 1, **dadurch gekennzeichnet**, daß Isonicotinamid-N-oxid mittels einem Dialkylsulfat, bevorzugt Dimethyl- oder Diethylsulfat bei 90 bis 105 °C zu einem N-Alkoxy-isonicotinamidsalz umgesetzt wird.

4. Verfahren gemäß Anspruch 1, **dadurch gekennzeichnet**, daß das gebildete N-Alkoxy-isonicotinamidsalz in wäßriger Lösung durch ein Natrium- oder Kaliumcyanid zum 2-Cyanoisonicotinamid umgesetzt wird.

5. Verfahren gemäß Anspruch 1, **dadurch gekennzeichnet**, daß zur sauren Hydrolyse des 2-Cyanoisonicotinamids Schwefelsäure oder Salzsäure verwendet und anschließend mit Ammoniaklösung ein pH im Bereich von 1 bis 4 eingestellt wird.

6. Verfahren nach Anspruch 1, **dadurch gekennzeichnet**, daß zur alkalischen Hydrolyse des 2-Cyanoisonicotinamids Natron- oder Kalilauge verwendet wird und anschließend mit Säure ein pH im Bereich von 1 bis 4 eingestellt wird.

7. 2-Cyanoisonicotinamid.

8. Verwendung des 2-Cyanoisonicotinamids als Zwischenprodukt für die Herstellung von Pyridin-2,4-dicarbonsäure.

## Claims

1. Process for the production of 2,4-pyridinedicarboxylic acid, characterised in that
a) isonicotinic acid amide is oxidised to the corresponding N-oxide
b) isonicotinamide N-oxide is reacted in the presence of an alkylating agent to yield the corresponding N-alkoxyisonicotinamide salt,
c) the salt is converted by an alkali cyanide in an aqueous solution to the 2-cyanoisonicotinamide
d) 2,4-pyridinedicarboxylic acid is obtained at high purity from this product by hydrolysis and subsequent pH adjustment to a pH in the range from 1 to 4.

2. Process according to claim 1, characterised in that isonicotinic acid amide is oxidised to the corresponding N-oxide in the presence of hydrogen peroxide in acetic or peracetic acid.

3. Process according to claim 1, characterised in that isonicotinamide N-oxide is reacted by means of a dialkyl sulphate, preferably dimethyl or diethyl sulphate, at 90 to 105° C to yield an N-alkoxyisonicotinamide salt.

4. Process according to claim 1, characterised in that the N-alkoxyisonicotinamide salt formed is converted by a sodium or potassium cyanide to the 2-cyanoisonicotinamide.

5. Process according to claim 1, characterised in that sulphric acid or hydrochlorid acid is used for acid hydrolysis of the 2-cyanoisonicotinamide and the pH is subsequently adjusted to the range from 1 to 4 with ammonia solution.

6. Process according to claim 1, characterised in that sodium or potassium hydroxide solution is used for alkaline hydrolysis of the 2-cyanoisonicotinamide and the pH is subsequently adjusted to the range from 1 to 4 with acid.

7. 2-Cyanoisonicotinamide

8. Use of 2-cyanoisonicotinamide as an intermediate for the production of 2,4-pyridinedicarboxylic acid.

## Revendications

1. Procédé de préparation de l'acide 2,4-pyridine-dicarboxylique, caractérisé en ce que :
a) l'isonicotinamide est oxydé en N-oxyde correspondant;
b) le N-oxyde de l'isonicotinamide est transformé, en présence d'un agent alcoylant, en un sel de N-alcoxyisonicotinamide;
c) le sel est transformé par un cyanure alcalin en solution aqueuse en 2-cyanoisonicotinamide et
d) a partir de ce produit, on obtient l'acide 2,4-pyridinedicarboxylique sous forme très pure, par hydrolyse et ensuite ajustement du pH à une valeur dans l'intervalle de 1 a 4.

2. Procédé suivant la revendication 1, caractérisé en ce que l'isonicotinamide est oxydé en N-oxyde correspondant en présence de peroxyde d'hydrogène dans de l'acide acétique ou dans de l'acide peracétique.

3. Procédé suivant la revendication 1, caractérisé en ce que le N-oxyde de l'isonicotinamide est transformé en un sel de N-alcoxyisonicotinamide au moyen d'un dialcoylsulfate, de préférence, le diméthyl- ou le diéthylsulfate, à 90 à 105°C.

4. Procédé suivant la revendication 1, caractérisé en ce que le sel de N-alcoxyisonicotinamide formé est transformé en 2-cyanoisonicotinamide, en solution aqueuse, par un cyanure de sodium ou de potassium.

5. Procédé suivant la revendication 1, caractérisé en ce que l'on utilise de l'acide sulfurique ou de l'acide chlorhydrique pour l'hydrolyse acide du 2-cyanoisonicotinamide et ensuite, que l'on ajuste le pH dans l'intervalle de 1 à 4 avec une solution d'ammoniaque.

6. Procédé suivant la revendication 1, caractérisé en ce que l'on utilise de la lessive de soude ou de potasse pour l'hydrolyse alcaline du 2-cyanoisonicotinamide et ensuite, que l'on ajuste le pH dans l'intervalle de 1 à 4 avec un acide.

7. 2-Cyanoisonicotinamide.

8. Utilisation du 2-cyanoisonicotinamide comme produit intermédiaire pour la préparation de l'acide 2,4-pyridinedicarboxylique.
